# FASCICULE DE BREVET EUROPEEN

(11) **EP 0 608 178 B1**
(45) Date de publication et mention de la délivrance du brevet: **31.07.1996**
(21) Numéro de dépôt: 94400114.8
(22) Date de dépôt: 19.01.1994
(51) Int. Cl.: C07J 5/00, C07J 71/00

(54) **Nouveau procédé de préparation de stéroides 16alpha-méthylés**
Neues Verfahren zur 16-alpha Methylierung von Steroiden
New 16-alpha steroid-methylation process

(30) Priorité: 20.01.1993 FR 9300519
(43) Date de publication de la demande: 27.07.1994
(73) Titulaire: ROUSSEL UCLAF, F-93230 Romainville (FR)
(72) Inventeur: Roussel, Patrick, F-94320 Thiais (FR); Vivat, Michel, F-77400 Lagny sur Marne (FR)
(74) Mandataire: Vieillefosse, Jean-Claude

(56) Documents cités:
- EP-A- 0 165 037
- EP-A- 0 574 318
- WO-A-87/07612
- FR-A- 2 318 647
- GB-A- 1 277 266
- US-A- 3 080 393
- US-E- R E28 369

## Description

La présente invention concerne un nouveau procédé de préparation de stéroïdes 16α-méthylés.

L'invention a ainsi pour objet un procédé de préparation des composés de formule (I) : 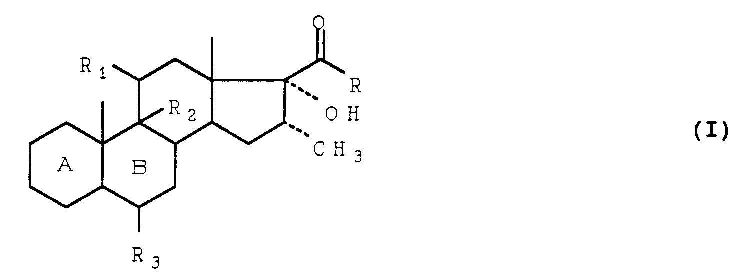
dans laquelle les cycles A et B représentent un reste : 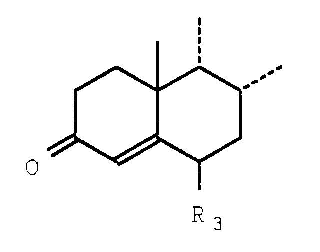
dans lequel la fonction cétone en position 3 est éventuellement protégée sous forme de cétal, de thiocétal, d'hémithiocétal ou d'éther d'énol, ou un reste : 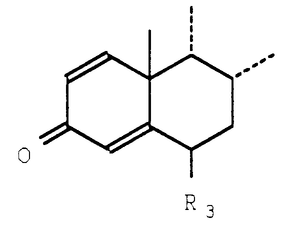
R représente un radical méthyle ou un radical -CH₂-OR', dans lequel R' représente un atome d'hydrogène ou un reste éther ou un reste ester, R₁ et R₂ forment ensemble une seconde liaison, ou R₁ et R₂ forment ensemble un époxyde en position β, ou R₁ représente un atome d'hydrogène, une fonction cétone ou une fonction hydroxy en position α ou β, libre ou protégée sous forme d'éther ou d'ester et R₂ représente un atome d'hydrogène, ou R₁ représente un atome d'hydrogène et R₂ représente une fonction hydroxy en position α, ou R₁ représente une fonction hydroxy en position β, libre ou protégée sous forme d'éther ou d'ester et R₂ représente un atome de fluor ou de brome en position α, et R₃ représente un atome d'hydrogène ou un atome de fluor ou un radical méthyle, en position α ou β, caractérisé en ce que l'on traite un composé de formule (II) : 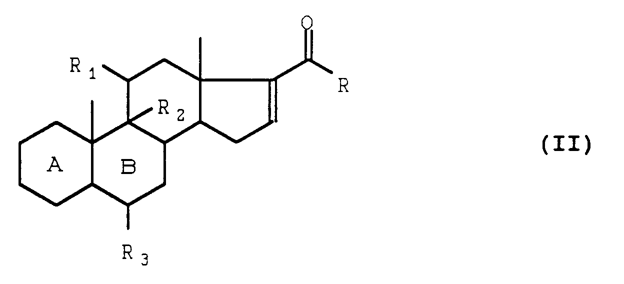
dans laquelle A, B, R, R₁, R₂ et R₃ ont la signification déjà indiquée, par un agent de méthylation en présence d'un catalyseur à base de cuivre, puis hydrolyse l'énolate 16α-méthylé ainsi pour obtenir l'énol correspondant formé et fait agir sur cet énol un agent d'oxydation, pour obtenir le composé attendu.

Lorsque la fonction cétone en 3 est protégée sous forme de cétal, thiocétal ou hémithiocétal, il s'agit de préférence d'un groupement de formule : 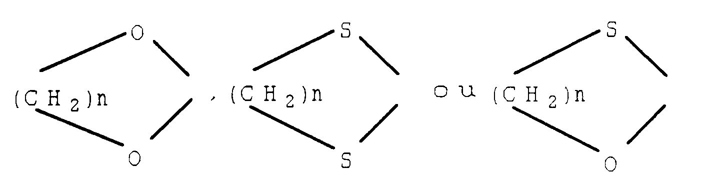
dans lequel n est égal à 2 ou 3 et tout particulièrement d'un groupement éthylènedioxy ou éthylènedithio.

Lorsque la fonction cétone en 3 est protégée sous forme d'éther d'énol, il s'agit de préférence d'un groupement alkoxy ou alkoxyalkoxy renfermant de 1 à 8 atomes de carbone et plus particulièrement d'un groupement méthoxy, éthoxy, éthoxyéthoxy ou 1-éthoxyéthoxy, les cycles A et B comportant alors un système de doubles liaisons Δ 3,5.

Lorsque R' représente un reste éther, il peut s'agir de tout reste connu de l'homme du métier et notamment d'un radical alkyle renfermant de 1 à 6 atomes de carbone, par exemple méthyle, éthyle ou propyle, d'un radical tétrahydropyranyle, ou d'un reste d'éther silylé, par exemple trialkylsilyle tel que triméthyl ou diméthylterbutylsilyle, triarylsilyle tel que triphénylsilyle ou diarylalkylsilyle tel que diphénylterbutylsilyle.

Lorsque R' représente un reste ester, il peut s'agir de tout reste connu de l'homme du métier et notamment d'un radical acyle renfermant de 1 à 8 atomes de carbone, par exemple formyle, acétyle, propionyle, butyryle, valéryle ou benzoyle.

Lorsque R₁ représente une fonction hydroxy protégée sous forme d'éther ou d'ester, il s'agit de l'un des restes éthers ou esters mentionnés plus haut pour R', étant entendu que ces restes ne sont pas nécessairement identiques.

L'invention a notamment pour objet un procédé tel que défini précédemment, caractérisé en ce que l'on utilise au départ un composé de formule (II) dans laquelle les cycles A et B représentent un reste : 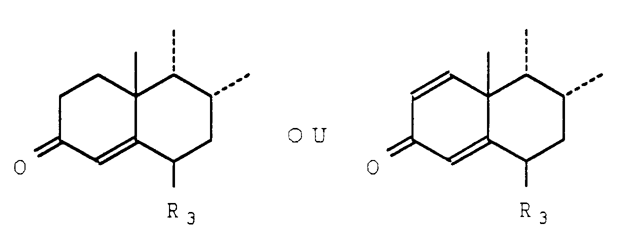
dans lequel R₃ est défini comme précédemment et la fonction cétone en position 3 est libre, le dernier représenté étant plus particulièrement préféré.

L'invention a aussi notamment pour objet un procédé tel que défini précédemment, caractérisé en ce que l'on utilise au départ un composé de formule (II) dans laquelle R₁ et R₂ forment ensemble une seconde liaison, ou R₁ et R₂ forment ensemble un époxyde en position β, ou R₁ représente une fonction hydroxy en position β, libre ou protégée sous forme d'éther ou d'ester et R₂ représente un atome de fluor en position α, et R₃ représente un atome d'hydrogène, un atome de fluor ou un radical méthyle et, de préférence, un atome d'hydrogène.

L'agent de méthylation utilisé peut être un dérivé méthylé du cuivre, par exemple CH₃Cu, (CH₃)₂ CuMg, (CH₃)₂ CuLi ou, de préférence, un chlorure, bromure ou iodure de méthylmagnésium, utilisé en présence d'un catalyseur à base de cuivre. Le catalyseur peut être un sel tel que l'acétate, le propionate ou le chlorure cuivrique, le chlorure, le bromure, l'iodure ou le cyanure cuivreux, ou encore un complexe, par exemple l'acétylacétonate de cuivre, le bromure diméthylsulfure cuivreux ou encore le tri-nbutylphosphine chlorure cuivreux ou tout autre complexe du même type connu de l'homme du métier. L'acétate et le proprionate cuivrique sont tout particulièrement préférés.

On opère au sein d'un solvant qui est de préférence un éther tel que le tétrahydrofuranne, le dioxane, le terbutylméthyléther, le diméthoxyéthane, l'éther éthylique, le t-butyl méthyl éther, le di n-butyl éther. Le tétrahydrofuranne est plus particulièrement préféré.

On opère avantageusement à une température de 0 à -30°C et, de préférence, à -20°C.

L'hydrolyse de l'énolate 16α-méthylé est de préférence effectuée sous gaz inerte, en versant la solution réactionnelle dans une solution aqueuse d'un phosphate monoalcalin, par exemple de sodium ou de potassium, ou dans un tampon de pH faiblement acide, notamment un tampon phosphate, ou, plus généralement, dans un agent acide faible tel que l'acide acétique, propionique ou butyrique, ou encore dans une solution aqueuse de chlorure ou d'acétate d'ammonium. Un tampon phosphate est plus particulièrement préféré.

L'hydrolyse est de préférence suivie d'un traitement oxydant, afin de transformer les ions cuivreux présents dans le mélange en ions cuivriques, dans le but d'en faciliter la précipitation. Les réactifs oxydants classiques connus de l'homme du métier pour réaliser cette transformation, peuvent être utilisés. A titre d'exemple, on peut indiquer que l'eau oxygénée convient particulièrement. La précipitation est rendue plus aisée encore par addition d'un sel alcalin, par exemple le sulfate ou le chlorure de sodium, afin de saturer le milieu.

L'oxydation de l'énol selon le procédé de l'invention est effectuée par un agent d'oxydation classique choisi dans le groupe constitué par l'eau oxygénée, utilisée seule ou associée soit à un métal de transition, notamment le titane, le manganèse ou le tungstène, soit à l'acétone ou un dérivé, notamment l'hexachloro ou l'hexafluoro acétone; le permanganate de potassium, activé ou non par des métaux tels que le cuivre; et un agent d'époxydation choisi dans le groupe constitué par un dioxirane, par exemple le diméthyldioxirane, un hydroperoxyde, par exemple l'hydroperoxyde de terbutyle, et un peracide, par exemple l'acide perphtalique, l'acide perbenzoïque, l'acide n-chloroperbenzoïque, l'acide peracétique, l'acide trifluoroperacétique ou l'acide permaléique. L'oxydation par un peracide est particulièrement préférée.

On opère de préférence sous gaz inerte, au sein d'un solvant halogéné, tel que le chlorure de méthylène, le chloroforme, le dichloroéthane, d'un éther tel que l'éther éthylique, le tétrahydrofuranne ou le dioxane, d'un solvant aromatique tel que le toluène, ou d'un ester, tel que l'acétate d'éthyle, ou encore de l'acétonitrile, le cas échéant en présence d'un cosolvant, par exemple un alcanol tel que le méthanol, l'éthanol, l'isopropanol ou, de préférence, le terbutanol, à une température de préférence comprise entre -10 et +10°C.

D'une manière plus particulièrement préférée, l'invention a pour objet un procédé tel que défini plus haut, caractérisé en ce que l'on effectue l'oxydation par l'acide perphtalique au sein du tétrahydrofuranne.

Des procédés de préparation de composés de formule (I) ont déjà été décrits, par exemple dans la demande WO 87/07612. Le procédé de cette demande consiste à méthyler un dérivé 16 insaturé par un agent de méthylation en présence d'un catalyseur à base de cuivre et d'un agent de silylation, dans le but d'isoler intermédiairement l'éther d'énol de formule : 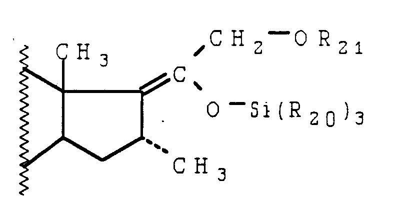 lequel est ensuite traité par un peracide pour former l'époxyde 17α-20 de formule : 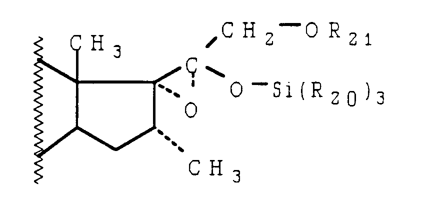 qui est enfin hydrolysé par un acide ou une base. Ce procédé requiert donc le passage par un époxyde issu d'un énol stabilisé, sous forme d'éther silylé, lequel doit ensuite être hydrolysé (voir aussi EP-A-165,037-pour la même séquence de réactions). Les documents FR-2,318,647 et US-Re-28,369 décrivent un procédé correspondant dans lequel l'énol est stabilisé par esterification, et US-A-3,080,393 décrit un tel procédé dans lequel l'énol est stabilisé par salification Le document EP-A-574,318 décrit un procédé correspondant à celui de l'invention, dans lequel est utilisée la barbotage de l'oxygène moleculaire en presence d'un agent reducteur et completant de cuivre.

Le procédé de la présente invention ne requiert l'isolement d'aucun intermédiaire, le produit issu de la réaction de méthylation étant directement hydrolysé puis oxydé dans des conditions jamais envisagées à ce jour et particulièrement intéressantes d'un point de vue industriel. Ce procédé ne requiert donc pas le passage par une quelconque forme stabilisée de l'énol intermédiaire, ni d'hydrolyse finale pour isoler le produit. C'est d'ailleurs la première fois, à la connaissance de la demanderesse, qu'est ainsi réalisée directement l'oxydation d'un tel énol.

On peut encore citer le brevet anglais 2 001 990 qui décrit également un procédé de préparation de composés de formule (I) qui consiste comme ci-dessus à méthyler un dérivé 16-insaturé, puis à préparer et isoler l'hydroperoxyde de formule : 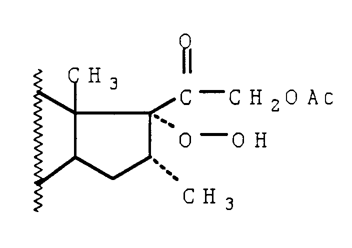
lequel est ensuite réduit par le zinc dans l'acide acétique ou par un iodure alcalin dans une cétone aliphatique.

Il s'agit donc d'un procédé qui, dans son principe comme dans les intermédiaires qu'il met en oeuvre, est différent de celui de la présente invention.

Les composés de formule (II) sont connus et décrits, par exemple, dans les brevets US 2 345 711, 2 883 400, 2 963 496, 2 966 504, 2 975 197, 3 029 233, 3 210 341, 3 377 343, 3 839 369, 3 976 638, 4 031 080, 4 277 409, 4 929 395, le brevet allemand 2 207 420, le brevet néerlandais 69 02 507 ou les brevets belges 539 498, 540 478, 711 016, ou peuvent être aisément préparés à partir des composés décrits dans ces brevets, par des procédés connus de l'homme du métier.

Les composés 16α-méthyl de formule (I) sont connus pour posséder une activité anti-inflammatoire et cette formule englobe notamment la dexaméthasone ses dérivés fluméthasone (6α-fluoro), paraméthasone (6α-fluoro 9H) et des précurseurs possibles (Δ 9,11,9α-OH ou 9,11-époxy).

Les exemples suivants illustrent l'invention sans toutefois la limiter.

### EXEMPLE 1 : 9β, 11β-époxy 16α-méthyl 17α-hydroxy 21-acétyloxy pregna 1,4-diène 3,20-dione

On mélange sous gaz inerte, 0,2 g d'acétate cuivrique monohydraté, 7,66 g de 9β,11β-époxy 21-acétyloxy pregna 1,4,16(17)-triène 3,20-dione et 100 cm³ de tétrahydrofuranne anhydre. Après 10 mn à 20°C, on refroidit à -20°C et ajoute en 2 h 8,8 cm³ d'une solution 3M de chlorure de méthylmagnésium dans le tétrahydrofuranne. On poursuit l'agitation pendant 15 mn, puis refroidit à -30°C. On verse lentement le mélange, sous gaz inerte, sur 80 cm³ d'un mélange à 0°C d'acide phosphorique (1 M) et de soude (1,35 M). On laisse le mélange se réchauffer sous agitation puis, après 40 mn, on ajoute à +15°C, 1 cm³ d'eau oxygénée (1 M). Après 1 h sous agitation à +15, +20°C, on ajoute 8 g de chlorure de sodium et poursuit l'agitation pendant 10 mn. On décante sous gaz inerte, extrait la phase aqueuse au tétrahydrofuranne, puis lave la phase organique avec 10 cm³ du mélange acide phosphorique-soude ci-dessus. On ajoute 2 g de chlorure de sodium, agite le mélange pendant quelques minutes puis décante. On ajoute 2 g de chlorure de sodium à la phase organique puis agite et décante à nouveau.

On mélange par ailleurs, 8 g d'anhydride phtalique et 5 cm³ d'eau oxygénée 1M. On agite pendant 1 h à température ambiante puis ajoute 5 cm³ de tétrahydrofuranne. On agite pendant 1 h 15 mn puis rajoute 3 cm³ de tétrahydrofuranne. On agite pendant 1 h puis rajoute à nouveau 5 cm³ de tétrahydrofuranne. On maintient sous agitation pendant encore 1 heure.

On ajoute 8 g de sulfate de sodium anhydre à la solution énolique préparée plus haut, à 0, +3°C et sous gaz inerte. On y ajoute la suspension de peracide préparée ci-dessus. Après 2 h sous agitation à +5°C environ, on neutralise par addition de 9 g de bicarbonate de sodium dans 90 cm³ d'eau. On ajoute 8 g de chlorure de sodium, agite pendant 15 mn puis décante et réextrait la phase aqueuse à l'acétate d'éthyle. On lave les phases organiques réunies à l'eau salée puis sèche et concentre sous pression réduite à 30°C environ. On obtient 6,6 g de produit brut. On chromatographie sur silice 1/5 du produit brut, en éluant au mélange chlorure de méthylèneéther éthylique (7-3). On obtient 1,321 g de produit attendu soit un rendement de 80 %.

### Spectre IR (CHCl₃)

Absorptions à 3610 cm⁻¹ (OH) ; 1747-1728 cm⁻¹ (C=O) ; 1663-1625-1607 cm⁻¹ (Δ1,4 3-one).

### Spectre RMN (CDCl₃-300 MHz-ppm)

0,88 (d) : CH₃ en 16 ; 0,93 : CH₃ en 18 ; 1,44 : CH₃ en 19 ; 2,16 : CH₃ de -OAc ; 2,94 : -OH ; 3,21 : H en 11 ; 4,69 (d) et 5,04 (d) : 2H en 21 ; 6,14 : H en 4 ; 6,20 : H en 2 ; 6,62 : H en 1.

### EXEMPLE 2 : 16α-méthyl 17α-hydroxy 21-acétyloxy pregna 1,4,9(11)-triène 3,20-dione

On opère de manière analogue à celle décrite à l'exemple 1, en utilisant 0,02 g d'acétate cuivrique monohydraté et 0,733 g de 16α-méthyl 21-acétyloxy pregna 1,4,9(11)-16(17)-tétraène 3,20-dione dans 10 cm³ de tétrahydrofuranne, puis 1 cm³ d'une solution 3M dans le tétrahydrofuranne de chlorure de méthylmagnésium, 8 cm³ de mélange acide phosphorique-soude et 0,1 cm³ d'eau oxygénée.

Le peracide est préparé comme à l'exemple 1, à partir de 0,59 g d'anhydride phtalique.

On obtient après purification par chromatographie sur silice en éluant au mélange chlorure de méthylène-éther éthylique (7-3), 0,4 g de produit attendu.

### Spectre RMN (CDCl₃-300 MHz-ppm)

0,75 : CH₃ en 18 ; 0,94 (d) : CH₃ en 16 ; 1,40 : CH₃ en 19 ; 2,17 : CH₃ de -OAc ; 4,82 (d)-4,99 (d) : 2H en 21 ; 5,57 : H en 11 ; 6,05 : H en 4 ; 6,28 : H en 2 ; 7,19 : H en 1.

### EXEMPLE 3 : 9α-fluoro 11β-hydroxy 16α-méthyl 17α-hydroxy 21-acétyloxy pregna 1,4-diène 3,20-dione

On opère de manière analogue à celle décrite à l'exemple 1, en utilisant 0,02 g d'acétate cuivrique monohydraté et 0,805 g de 9α-fluoro 11β-hydroxy 21-acétyloxy pregna 1,4,9(11)-triène 3,20-dione dans 15 cm³ de tétrahydrofuranne, puis 2 cm³ d'une solution 3M de chlorure de méthylmagnésium dans le tétrahydrofuranne, 12 cm³ de mélange acide phosphorique-soude et 0,1 cm³ d'eau oxygénée.

Le peracide est préparé comme à l'exemple 1, à partir de 0,59 g d'anhydride phtalique.

On obtient après purification par chromatographie sur silice en éluant au mélange chlorure de méthylène-acétate d'éthyle (7-3), 0,25 g de produit attendu.

### Spectre RMN (CDCl₃-300 MHz-ppm)

0,93 (d) : CH₃ en 16 ; 1,07 : CH₃ en 18 ; 1,57 : CH₃ en 19 ; 2,17 : CH₃ de -OAc ; 3,39 : OH en 11β et 17α ; 4,38 : H en 11 **;** 4,92 : 2H en 21 ; 6,11 : H en 4 ; 6,33 : H en 2 ; 7,25 : H en 1.

### EXEMPLE 4 : 9α-fluoro 11β,21-diacétyloxy 16α-méthyl 17α-hydroxy pregna 1,4-diène 3,20-dione

On opère de manière analogue à celle décrite à l'exemple 1, en utilisant 0,02 g d'acétate cuivrique monohydraté et 0,889 g de 9α-fluro 11β,21-diacétyloxy pregna 1,4,9(11)-triène 3,20-dione dans 10 cm³ de tétrahydrofuranne, puis 1 cm³ d'une solution 3M de chlorure de méthylmagnésium dans le tétrahydrofuranne, 8 cm³ de mélange acide phosphorique-soude et 0,1 cm³ d'eau oxygénée.

Le peracide est préparé comme à l'exemple 1, à partir de 0,59 g d'anhydride phtalique.

On obtient après purification par chromatographie sur silice en éluant au mélange chlorure de méthylène-éther éthylique (7-3), 0,634 g de produit attendu.

### Spectre RMN (CDCl₃-300 MHz-ppm)

0,93 (d) : CH₃ en 16 ; 0,93 : CH₃ en 18 ; 1,58 : CH₃ en 19 ; 2,13-2,15 : CH₃ de -OAc en 21 et 11β ; 2,74 : OH ; 4,71 (d)-4,99 (d) : 2H en 21 ; 5,42 (m) : H en 11α ; 6,11 : H en 4 ; 6,33 : H en 2 ; 6,82 : H en 1.

### EXEMPLE 5 : 9β,11β-époxy 16α-méthyl 17α-hydroxy pregna 1,4-diène 3,20-dione

On opère de manière analogue à celle décrite à l'exemple 1, en utilisant 0,02 g d'acétate cuivrique monohydraté et 0,649 g de 9β,11β-époxy pregna 1,4,16(17)-triène 3,20-dione dans 10 cm³ de tétrahydrofuranne, puis 0,9 cm³ d'une solution 3M de chlorure de méthylmagnésium dans le tétrahydrofuranne, 8 cm³ de mélange acide phosphorique-soude et 0,1 cm³ d'eau oxygénée.

Le peracide est préparé comme à l'exemple 1, à partir de 0,59 g d'anhydride phtalique.

On obtient après purification par chromatographie sur silice en éluant au mélange chlorure de méthylène-éther éthylique (7-3) 0,234 g de produit attendu.

### Spectre RMN (CDCl₃-300 MHz-ppm)

0,88 (d) : CH₃ en 16 ; 1,01 : CH₃ en 18 ; 1,44 : CH₃ en 19 ; 2,24 : CH₃ en 21 ; 3,05 : OH en 17 ; 3,21 : H en 11α ; 6,15 : H en 4 ; 6,18 : H en 2 ; 6,60 : H en 1.

### EXEMPLE 6 : 9β, 11β-époxy 16α-méthyl 17α-hydroxy 21-acétyloxy pregna 1,4-diène 3,20-dione

On mélange sous gaz inerte, 3,06 g de 9β,11β-époxy 21-acétyloxy pregna 1,4,16(17)-triène 3,20-dione et 0,08 g d'acétate cuivrique monohydraté dans 33 cm³ de tétrahydrofuranne. On refroidit et introduit à -20°C en 1 h, 4 cm³ d'une solution 3M de chlorure de méthylmagnésium dans le tétrahydrofuranne. On poursuit l'agitation pendant 1 h puis refroidit à -30°C. On verse lentement le mélange sur 30 cm³ d'un mélange refroidi à 0°C d'acide phosphorique et de soude (1M/1,35 M). On laisse le mélange se réchauffer sous agitation, puis ajoute à +15°C, 0,4 cm³ d'eau oxygénée 1M. Après 1 h à +15°/+20°C, on ajoute 4 g de chlorure de sodium. On agite pendant 10 mn puis décante sous gaz inerte. On extrait la phase aqueuse au tétrahydrofuranne puis lave la phase organique par 5 cm³ du mélange acide phosphorique-soude ci-dessus, ajoute 1 g de chlorure de sodium puis décante l'eau et complète à 50 cm³. On prélève sous gaz inerte 10 cm³ de solution, concentre à sec puis reprend par du chlorure de méthylène. On refroidit à 0°C, puis ajoute 0,1 cm³ d'hexachloroacétone puis 0,6 cm³ d'eau oxygénée 1M. On agite à 0, +5°C pendant 15 h, puis ajoute un peu d'eau saturée de chlorure de sodium, décante la phase organique, la sèche et la concentre à sec. On chromatographie le résidu sur silice en éluant au mélange chlorure de méthylène-éther éthylique (7-3) et obtient 0,047 g de produit attendu.

### Spectre RMN (CDCl₃-300 MHz-ppm)

0,88 (d) : CH₃ en 16 ; 0,93 : CH₃ en 18 ; 1,44 : CH₃ en 19 ; 2,16 : CH₃ de -OAc ; 2,94 : OH ; 3,21 : H en 11α ; 4,69 (d)-5,04 (d) : 2H en 21 ; 6,14 : H en 4 ; 6,20 : H en 2 ; 6,62 : H en 1.

### EXEMPLE 7 : 9β, 11β-époxy 16α-méthyl 17α-hydroxy 21-acétyloxy pregna 1,4-diène 3,20-dione

On opère de manière identique à celle décrite à l'exemple 6 jusqu'au prélèvement sous gaz inerte de 10 cm³ de solution issue de l'hydrolyse. On concentre à sec puis reprend au chlorure de méthylène. On ajoute un mélange de 4 g de permanganate de potassium, 2 g de sulfate de cuivre (5H₂O) et 0,2 cm³ d'eau puis 1 cm³ d'alcool terbutylique. On agite pendant 3 h à 20°C, filtre, sèche et concentre à sec. On chromatographie le résidu sur silice en éluant aux mélanges cyclohexane-acétate d'éthyle (1-1) puis chlorure de méthylène-éther éthylique (7-3). On obtient 0,072 g de produit attendu.

### Spectre RMN (CDCl₃-300 MHz-ppm)

0,88 (d) : CH₃ en 16 ; 0,93 : CH₃ en 18 ; 1,44 : CH₃ en 19 ; 2,16 : CH₃ de -OAc ; 2,94 : OH ; 3,21 : H en 11α ; 4,69 (d)-5,04 (d) : 2H en 21 ; 6,14 : H en 4 ; 6,20 : H en 2 ; 6,62 : H en 1.

## Revendications

1. Procédé de préparation des composés de formule (I) : 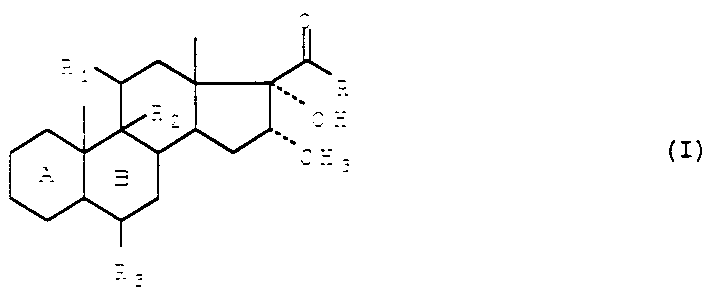 dans laquelle les cycles A et B représentent un reste : 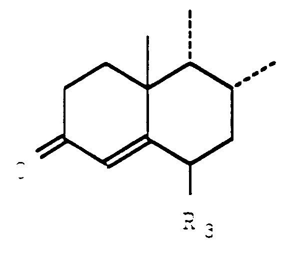 dans lequel la fonction cétone en position 3 est éventuellement protégée sous forme de cétal, de thiocétal, d'hémithiocétal ou d'éther d'énol, ou un reste : 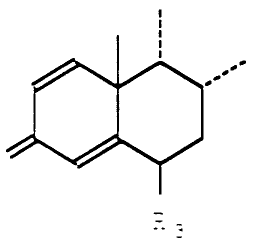 R représente un radical méthyle ou un radical -CH₂-OR', dans lequel R' représente un atome d'hydrogène ou un reste éther ou un reste ester, R₁ et R₂ forment ensemble une seconde liaison, ou R₁ et R₂ forment ensemble un époxyde en position β, ou R₁ représente un atome d'hydrogène, une fonction cétone ou une fonction hydroxy en position α ou β, libre ou protégée sous forme d'éther ou d'ester et R₂ représente un atome d'hydrogène, ou R₁ représente un atone d'hydrogène et R₂ représente une fonction hydroxy en position α, ou R₁ représente une fonction hydroxy en position β, libre ou protégée sous forme d'éther ou d'ester et R₂ représente un atome de fluor ou de brome en position α, et R₃ représente un atome d'hydrogène ou un atome de fluor ou un radical méthyle, en position α ou β, caractérisé en ce que l'on traite un composé de formule (II) : 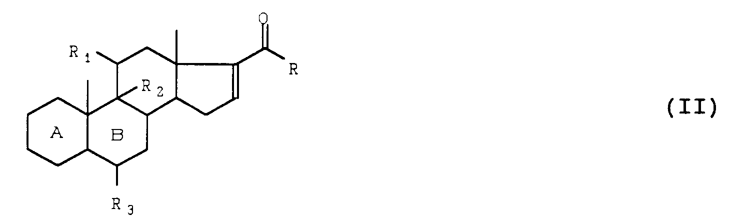 dans laquelle A, B, R, R₁, R₂ et R₃ ont la signification déjà indiquée, par un agent de méthylation en présence d'un catalyseur à base de cuivre, puis hydrolyse l'énolate 16α-méthylé ainsi formé pour obtenir l'énol correspondant et fait agir sur cet énol un agent d'oxydation choisi dans le groupe constitué par les peracides, l'eau oxygénée, les dioxiranes, les hydroperoxydes et le permanganate de potassium, pour obtenir le composé attendu.

2. Procédé selon la revendication 1, caractérisé en ce que l'on utilise au départ un composé de formule (II) dans laquelle les cycles A et B représentent un reste : 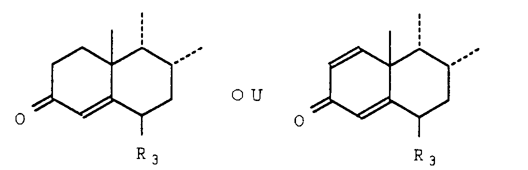 dans lequel R₃ est défini comme à la revendication 1 et la fonction cétone en position 3 est libre.

3. Procédé selon la revendication 1 ou 2, caractérisé en ce que l'on utilise au départ un composé de formule (II) dans laquelle R₁ et R₂ forment ensemble une seconde liaison, ou R₁ et R₂ forment ensemble un époxyde en position β, ou R₁ représente une fonction hydroxy en position β, libre ou protégée sous forme d'éther ou d'ester et R₂ représente un atome de fluor en position α, et R₃ représente un atome d'hydrogène, un atome de fluor ou un radical méthyle.

4. Procédé selon l'une quelconque des revendications 1 à 3, caractérisé en ce que l'on utilise au départ un composé de formule (II) dans laquelle les cycles A et B représentent un reste :

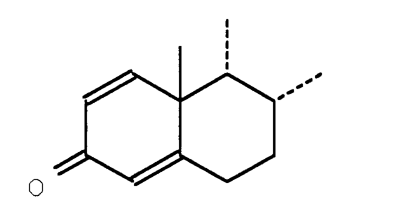

5. Procédé selon l'une quelconque des revendications 1 à 4, caractérisé en ce que l'agent de méthylation est un dérivé méthylé du cuivre ou un chlorure, bromure ou iodure de méthyl magnésium, utilisé en présence d'un catalyseur à base de cuivre.

6. Procédé selon l'une quelconque des revendications 1 à 5, caractérisé en ce que l'agent de méthylation est le chlorure, bromure ou iodure de méthyl magnésium, utilisé en présence d'acétate ou propionate cuivrique.

7. Procédé selon l'une quelconque des revendications 1 à 6, caractérisé en ce que l'agent d'hydrolyse est choisi dans le groupe constitué par un phosphate monoalcalin, le chlorure d'ammonium, l'acétate d'ammonium, un acide faible et un tampon de pH faiblement acide.

8. Procédé selon l'une quelconque des revendications 1 à 7, caractérisé en ce que l'hydrolyse est suivie d'un traitement oxydant des ions cuivreux présents dans le mélange.

9. Procédé selon l'une queconque des revendications 1 à 8, caractérisé en ce que l'agent d'oxydation est choisi dans le groupe constitué par les peracides.

10. Procédé selon l'une quelconque des revendications 1 à 9, caractérisé en ce que l'on effectue l'oxydation au sein d'un solvant halogéné, d'un éther, d'un solvant aromatique, d'un ester ou de l'acétonitrile, le cas échéant en présence d'un cosolvant.

11. Procédé selon l'une quelconque des revendications 1 à 10, caractérisé en ce que l'on effectue l'oxydation par l'acide perphtalique, au sein du tétrahydrofuranne.

## Claims

1. Preparation process for the compounds of formula (I): 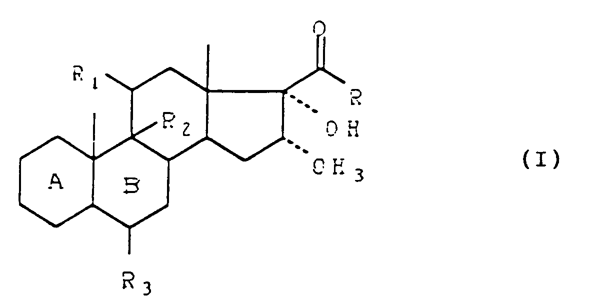 in which rings A and B represent a remainder: 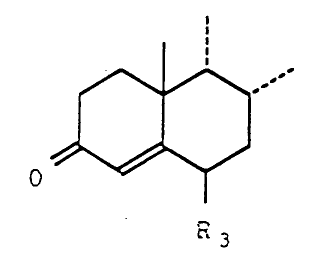 in which the ketone function in position 3 is optionally protected in the ketal, thioketal, hemithioketal or enol ether form, or a remainder: 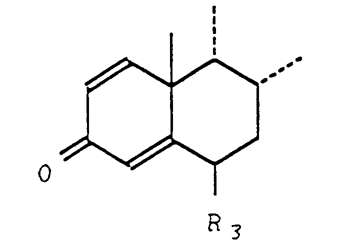 R represents a methyl radical or a -CH₂-OR' radical, in which R' represents a hydrogen atom or an ether remainder or an ester remainder, R₁ and R₂ together form a second bond, or R₁ and R₂ together form an epoxide in beta position, or R₁ represents a hydrogen atom, a ketone function or a hydroxy function in alpha or beta position, free or protected in the form of the ether or ester and R₂ represents a hydrogen atom, or R₁ represents a hydrogen atom and R₂ represents a hydroxy function in alpha position, or R₁ represents a hydroxy function in beta position, free or protected in the form of the ether or ester and R₂ represents a fluorine or bromine atom in alpha position, and R₃ represents a hydrogen atom or a fluorine atom or a methyl radical, in alpha or beta position, characterized in that a compound of formula (II): 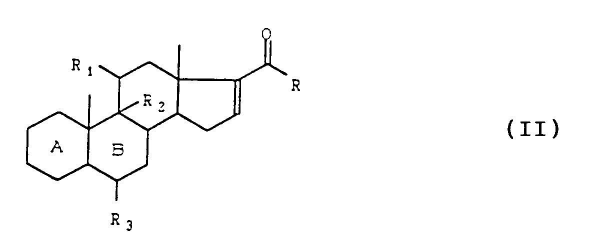 in which A, B, R, R₁, R₂ and R₃ have the meaning already indicated, is treated with a methylation agent in the presence of a copper-based catalyst, then the 16alpha-methylated enolate thus formed is hydrolyzed in order to obtain the corresponding enol and an oxidizing agent chosen from the group constituted by peracids, hydrogen peroxide, dioxiranes, hydroperoxides and potassium permanganate is reacted on this enol, in order to obtain the expected compound.

2. Process according to claim 1, characterized in that a compound of formula (II) is used at the start in which rings A and B represent a remainder: 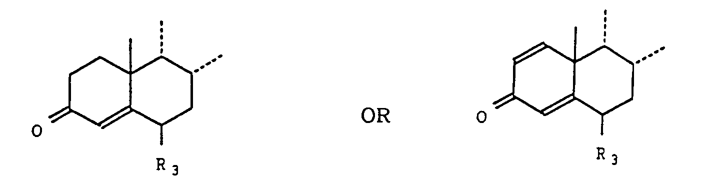 in which R₃ is defined as in claim 1 and the ketone function in position 3 is free.

3. Process according to claim 1 or 2, characterized in that a compound of formula (II) is used at the start in which R₁ and R₂ together form a second bond, or R₁ and R₂ together form an epoxide in beta position, or R₁ represents a hydroxy function in beta position, free or protected in the form of the ether or ester and R₂ represents a fluorine atom in alpha position, and R₃ represents a hydrogen atom, a fluorine atom or a methyl radical.

4. Process according to any one of claims 1 to 3, characterized in that a compound of formula (II) is used at the start in which rings A and B represent a remainder:

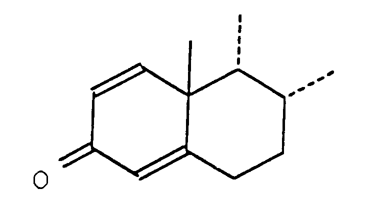

5. Process according to any one of claims 1 to 4, characterized in that the methylation agent is a methylated derivative of copper or a methyl magnesium chloride, bromide or iodide, used in the presence of a copper-based catalyst.

6. Process according to any one of claims 1 to 5, characterized in that the methylation agent is methyl magnesium chloride, bromide or iodide, used in the presence of cupric acetate or propionate.

7. Process according to any one of claims 1 to 6, characterized in that the hydrolysis agent is chosen from the group constituted by a monoalkaline phosphate, ammonium chloride, ammonium acetate, a weak acid and a buffer of weakly acidic pH.

8. Process according to any one of claims 1 to 7, characterized in that the hydrolysis is followed by an oxidizing treatment of the cupric ions present in the mixture.

9. Process according to any one of claims 1 to 8, characterized in that the oxidizing agent is chosen from the group constituted by peracids.

10. Process according to any one of claims 1 to 9, characterized in that the oxidation is carried out in a halogenated solvent, an ether, an aromatic solvent, an ester or acetonitrile, if appropriate in the presence of a cosolvant.

11. Process according to any one of claims 1 to 10, characterized in that the oxidation is carried out using perphthalic acid, in tetrahydrofuran.

## Patentansprüche

1. Verfahren zur Herstellung von Verbindungen der Formel (I) 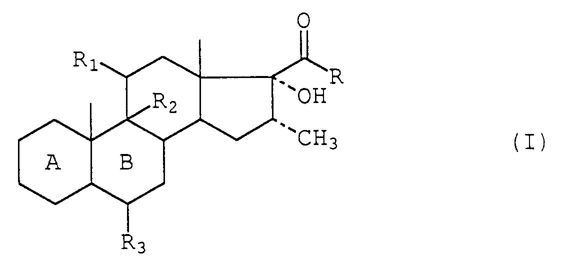 in der die Ringe A und B einen Rest 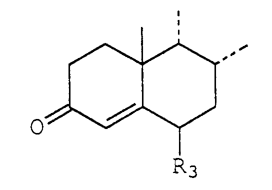 darstellen, worin die Ketonfunktion in Position 3 gegebenenfalls in Form von Ketal, Thioketal, Hemithioketal oder Enolether geschützt ist, oder einen Rest 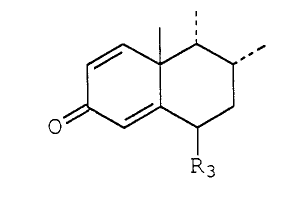 bedeuten,
R einen Methylrest oder einen Rest -CH₂-OR' darstellt, worin R' ein Wasserstoffatom, ein Etherrest oder ein Esterrest ist,
R₁ und R₂ zusammen eine zweite Bindung oder ein Epoxid in Position β bilden, oder R₁ stellt ein Wasserstoffatom, eine freie oder in Form von Ether oder Ester geschützte Ketonfunktion oder eine Hydroxyfunktion in Position α oder β dar und R₂ bedeutet ein Wasserstoffatom, oder R₁ stellt ein Wasserstoffatom dar und R₂ bedeutet eine Hydroxyfunktion in Position α, oder R₁ stellt eine freie oder in Form von Ether oder Ester geschützte Hydroxyfunktion in Position β dar und R₂ bedeutet ein Fluor- oder Bromatom in Position α, und
R₃ stellt ein Wasserstoffatom oder ein Fluoratom oder einen Methylrest in Position α oder β dar, dadurch gekennzeichnet, daß man eine Verbindung der Formel (II) 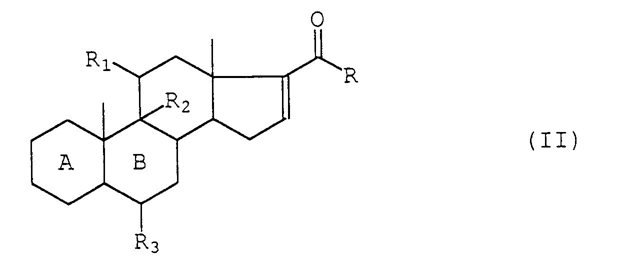 in der A, B, R, R₁, R₂ und R₃ die bereits angegebene Bedeutung besitzen, mit einem Methylierungsmittel in Anwesenheit eines Katalysators auf der Basis von Kupfer behandelt, anschließend das auf diese Weise gebildete 16α-Methyl-Enolat hydrolysiert, um das entsprechende Enol zu erhalten, und dieses Enol mit einem Oxidationsmittel zur Reaktion bringt, gewählt aus der durch die Persäuren, Wasserstoffperoxid, die Dioxirane, die Hydroperoxide und Kaliumpermanganat gebildeten Gruppe, um die erwartete Verbindung zu erhalten.

2. Verfahren nach Anspruch 1, dadurch gekennzeichnet, daß man als Ausgangsprodukt eine Verbindung der Formel (II) verwendet, in der die Ringe A und B einen Rest 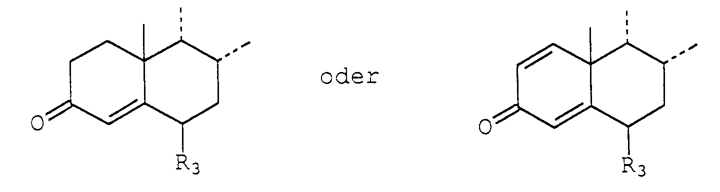 darstellen, worin R₃ wie in Anspruch 1 definiert und die Ketonfunktion in Position 3 frei ist.

3. Verfahren nach Anspruch 1 oder 2, dadurch gekennzeichnet, daß man als Ausgangsprodukt eine Verbindung der Formel (II) verwendet, in der R₁ und R₂ zusammen ein Epoxid in Position β bilden, oder R₁ stellt eine freie oder in Form von Ether oder Ester geschützte Hydroxyfunktion dar und R₂ bedeutet ein Fluoratom in Position α, und R₃ stellt ein Wasserstoffatom, ein Fluoratom oder einen Methylrest dar.

4. Verfahren nach irgendeinem der Ansprüche 1 bis 3, dadurch gekennzeichnet, daß man als Ausgangsprodukt eine Verbindung der Formel (II) verwendet, in der die Ringe A und B einen Rest 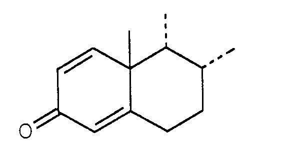 darstellen.

5. Verfahren nach irgendeinem der Ansprüche 1 bis 4, dadurch gekennzeichnet, daß das Methylierungsmittel ein Methylderivat von Kupfer oder ein Chlorid, Bromid oder Iodid von Methylmagnesium ist, verwendet in Anwesenheit eines Katalysators auf der Basis von Kupfer.

6. Verfahren nach irgendeinem der Ansprüche 1 bis 5, dadurch gekennzeichnet, daß das Methylierungsmittel das Chlorid, Bromid oder Iodid von Methylmagnesium ist, verwendet in Anwesenheit von Kupfer(II)-acetat oder Kupfer(II)-propionat.

7. Verfahren nach irgendeinem der Ansprüche 1 bis 6, dadurch gekennzeichnet, daß das Mittel zur Hydrolyse aus der durch Monoalkaliphosphat, Ammoniumchlorid, Ammoniumacetat, eine schwache Säure und einen Puffer mit schwach saurem pH-Wert gebildeten Gruppe gewählt wird.

8. Verfahren nach irgendeinem der Ansprüche 1 bis 7, dadurch gekennzeichnet, daß nach der Hydrolyse eine oxidierende Behandlung der in der Mischung vorliegenden Kupfer(I)-Ionen durchgeführt wird.

9. Verfahren nach irgendeinem der Ansprüche 1 bis 8, dadurch gekennzeichnet, daß das Oxidationsmittel aus der durch die Persäuren gebildeten Gruppe gewählt wird.

10. Verfahren nach irgendeinem der Ansprüche 1 bis 9, dadurch gekennzeichnet, daß man die Oxidation in einem halogenierten Lösungsmittel, einem Ether, einem aromatischen Lösungsmittel, einem Ester oder Acetonitril durchführt, gegebenenfalls in Anwesenheit eines Co-Lösungsmittels.

11. Verfahren nach irgendeinem der Ansprüche 1 bis 10, dadurch gekennzeichnet, daß man die Oxidation mit Perphthalsäure in Tetrahydrofuran durchführt.
